# EUROPÄISCHE PATENTSCHRIFT

(11) **EP 1 894 914 B1**
(45) Veröffentlichungstag und Bekanntmachung des Hinweises auf die Patenterteilung: **02.12.2009**
(21) Anmeldenummer: 07016720.0
(22) Anmeldetag: 27.08.2007
(51) Int. Cl.: C07C 68/02, C01B 31/28, C01D 3/06, C25B 1/26

(54) **Verfahren zur Herstellung von Diarylcarbonat**
Method for manufacturing diaryl carbonate
Procédé de fabrication de diarylcarbonates

(30) Priorität: 02.09.2006 DE 102006041465
(43) Veröffentlichungstag der Anmeldung: 05.03.2008
(73) Patentinhaber: Bayer MaterialScience AG, 51368 Leverkusen (DE)
(72) Erfinder: Bulan, Andreas, 40764 Langenfeld (DE); Ooms, Pieter, 47800 Krefeld (DE); Weber, Rainer, 51519 Odenthal (DE); Rechner, Johann, 47906 Kempen (DE); Buts, Marc, 2570 Duffel (BE); Vanden Eynde, Johan, 9052 Zwijnaarde (BE)

(56) Entgegenhaltungen:
- EP-A- 1 216 982
- WO-A-03/070639
- DE-A1-102004 041 777
- US-A- 4 025 405

## Beschreibung

Die Erfindung betrifft ein kombiniertes Verfahren zur Herstellung von Diarylcarbonat und Elektrolyse von Natriumchlorid-haltigem Prozessabwasser. Insbesondere besteht das Verfahren in der Verwertung von Natriumchlorid-haltigen Lösungen für das Diphenylcarbonat-Herstellungsverfahren (DPC-Verfahren)

Die Herstellung von Diarylcarbonaten (Diarylcarbonat) erfolgt üblicherweise durch ein kontinuierliches Verfahren, durch Herstellung von Phosgen und anschließende Reaktion von Monophenolen und Phosgen in einem inerten Lösungsmittel in Gegenwart von Alkali und einem Stickstoffkatalysator in der Grenzfläche.

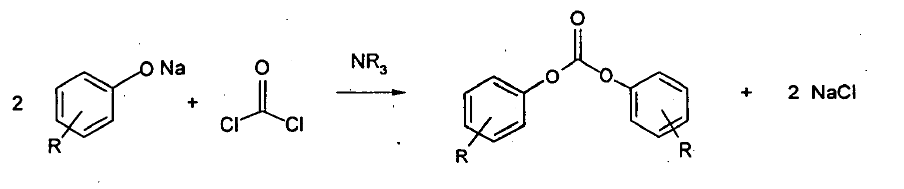

Die Herstellung von Diarylcarbonaten z.B. durch den Phasengrenzflächenprozess ist prinzipiell in der Literatur beschrieben, siehe z.B. in Chemistry and Physics of Polycarbonates, Polymer Reviews, H. Schnell, Vol. 9, John Wiley and Sons, Inc. (1964), S. 50/51.

In der US-A-4 016 190 wird ein Herstellungsverfahren von Diarylcarbonaten beschrieben, das bei Temperaturen >65°C betrieben wird. Der pH-Wert wird bei diesem Verfahren zunächst niedrig (pH 8 bis 9) und anschließend hoch (10 bis 11) eingestellt.

Optimierungen des Verfahrens durch Verbesserung der Durchmischung und Einhaltung eines engen Temperatur- und pH-Profils als auch Isolierung des Produkts werden in EP1219589 A1, EP1216981 A2, EP1216982 A2 und EP784048 A1 beschrieben.

Bei diesen bekannten Verfahren macht jedoch ein hoher Restphenolwert im Abwasser dieser Prozesse, welche die Umwelt belasten können und die Klärwerke vor eine erhöhte Abwasserproblematik stellen, aufwendige Reinigungsoperationen erforderlich. So beschreibt WO 03/070639 A1 eine Entfernung der organischen Verunreinigungen im Abwassers durch eine Extraktion mit Methylenchlorid.

Üblicherweise wird die Natriumchlorid-haltige Lösung von Lösungsmitteln und organischen Resten befreit und muss dann entsorgt werden.

Bekannt ist aber auch, dass die Reinigung der Natriumchlorid-haltigen Abwässer gemäß der EP 1200359 B1 (WO2000078682 A1) oder US-A-6340736 durch Ozonolyse erfolgen kann und dann zum Einsatz bei der Natriumchlorid-Elektrolyse geeignet ist. Nachteil der Ozonolyse ist, dass dieses Verfahren sehr kostenintensiv ist.

Gemäß der EP 541114 A2 wird ein Natriumchlorid-haltiger Abwasserstrom bis zur vollständigen Entfernung des Wassers eingedampft und das zurückbleibende Salz mit den organischen Verunreinigungen einer thermischen Behandlung unterzogen, wodurch die organischen Bestandteile zersetzt werden. Besonders bevorzugt ist dabei der Einsatz von Infrarotstrahlung. Nachteil des Verfahrens ist, dass das Wasser vollständig verdampft werden muss, so dass das Verfahren wirtschaftlich nicht durchführbar ist.

Gemäß der WO 03/70639 A1 wird das Abwasser aus einer DPC-Produktion durch Extraktion gereinigt und dann der Natriumchlorid-Elektrolyse zugeführt. Durch das beschriebene Verfahren können jedoch nur max. 26% des Natriumchlorids aus dem Abwasser der DPC-Produktion zurück gewonnen werden, da bei größeren Einsatzmengen das in die Elektrolyse mit dem Abwasser eingebrachte Wasser die Wasserbilanz der Natriumchlorid-Elektrolyse aus dem Gleichgewicht bringen würde.

Die Natriumchlorid-haltigen Lösungen, die bei der DPC-Produktion anfallen, haben typischerweise einen Natriumchlorid-Gehalt von 13 bis 17 Gew.%. Somit kann nie das in den Lösungen vorhandene gesamte Natriumchlorid wieder gewonnen werden. Bei einer Natriumchlorid-Konzentration von 17 Gew.-% gelingt bei der Standard-Natriumchlorid-Elektrolyse mit einer handelsüblichen Ionenaustauschermembran, die einen Wassertransport von 3,5 mol Wasser je mol Natrium aufzeigt, nur der Einsatz von ca. 23 % des Natriumchlorids aus dem Natriumchlorid-haltigen Lösungen. Selbst bei Aufkonzentrierung bis zu einer gesättigten Natriumchlorid-Lösung von ca. 25 Gew.-% gelänge nur eine Recyclingquote von 38 % des in der Natriumchlorid-haltigen Lösung enthaltenden Natriumchlorids. Ein vollständiges Recycling der Natriumchlorid-haltigen Lösung ist nicht bekannt geworden. Gemäß der WO 01/38419 kann die Natriumchlorid-haltige Lösung mittels thermischer Verfahren eingedampft werden, so dass eine hoch konzentrierte Natriumchlorid-Lösung der Elektrolysezelle zugeführt werden kann. Die Eindampfung ist jedoch energieintensiv und kostspielig.

Ausgehend vom oben geschilderten Stand der Technik ist die Aufgabe der Erfindung ein Diarylcarbonat-Herstellungsverfahren bereitzustellen, welches Produkte in hoher Reinheit und guter Ausbeute liefert und gleichzeitig eine Reduzierung der Umweltbelastung bzw. Abwasserproblematik in den Klärwerken durch maximierte Rückführung von Natriumchlorid aus Natriumchlorid-haltigen Prozessabwasserlösungen darstellt, die aus der Diarylcarbonat-Produktion erhalten werden.

Insbesondere sollte bei dem Recycling berücksichtigt werden, dass die Umsetzung von Natriumchlorid zu Chlor und Natronlauge und ggf. Wasserstoff mit minimalem Energieeinsatz und daher ebenfalls Resourcen-schonend erfolgen sollte.

Es wurde gefunden, dass die bei der kontinuierlichen Herstellung von Diarylcarbonaten durch Reaktion von Monophenolen und Phosgen in einem inerten Lösungsmittel in Gegenwart von Alkali und Aminkatalysator in der Phasengrenzfläche anfallende Natriumchlorid-haltige Abwasserlösungen ohne aufwendige Reinigung nach pH-Einstellung auf einen pH kleiner oder gleich 8 und einfacher Behandlung mit Aktivkohle direkt einer elektrochemischen Oxidation des enthaltenen Natriumchlorids zu Chlor, Natronlauge und gegebenenfalls Wasserstoff zugeführt werden können, wobei das Chlor zur Herstellung des Phosgens rückgeführt werden kann.

Gegenstand der Erfindung ist ein Verfahren zur Herstellung von Diarylcarbonat und Verarbeitung mindestens eines Teils der dabei anfallenden Alkalichlorid-haltigen Lösung in einer nachgeschalteten Alkalichlorid-Elektrolyse, umfassend die folgenden Schritte:
a) Herstellung von Phosgen durch Umsetzung von Chlor mit Kohlenmonoxid,
b) Umsetzung des gemäß Schritt a) gebildeten Phosgens mit wenigstens einem Monophenol in Gegenwart einer Alkali-haltigen Base, insbesondere einer Natrium-haltigen Base, ggf. basischem Katalysator und ggf. organischem Lösungsmittel zu einem Diarylcarbonat und einer Alkalichlorid-haltigen, insbesondere einer Natriumchlorid-haltigen Lösung,
c) Abtrennung und Aufarbeitung des in Schritt b) gebildeten Diarylcarbonats,
d) Abtrennung der gemäß Schritt c) verbliebenen Alkalichlorid-haltigen Lösung von Lösungsmittelresten und ggf. Katalysatorresten insbesondere durch Strippen der Lösung mit Wasserdampf und Behandlung mit Adsorbentien, insbesondere mit Aktivkohle,
e) elektrochemische Oxidation wenigstens eines Teils der Alkalichlorid-haltigen Lösung aus d) unter Bildung von Chlor, Alkalilauge und ggf. Wasserstoff,
   dadurch gekennzeichnet, dass bei der Abtrennung d) der Lösung vor Behandlung mit Adsorbentien die Lösung auf einen pH-Wert kleiner oder gleich 8 eingestellt wird und
f) wenigstens ein Teil des gemäß Schritt e) hergestellten Chlors in die Herstellung von Phosgen gemäß Schritt a) zurückgeführt wird und/oder
g) wenigstens ein Teil des gemäß Schritt e) hergestellten Alkalilauge in die Herstellung von Diarylcarbonat gemäß Schritt b) zurückgeführt wird.

Bevorzugt wird die Alkalichlorid-haltige Lösung des Reaktionsabwassers aus Schritt b) oder wenigstens teilweise mit Waschwasser aus der Aufarbeitung c) des Diarylcarbonats vereinigt eingesetzt.

Besonders geeignete Monophenole zum Einsatz in dem neuen Verfahren sind Phenole der Formel (I) 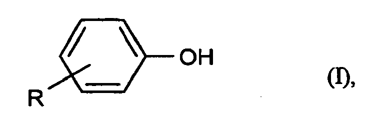 worin
- R: Wasserstoff, Halogen oder ein verzweigter oder unverzweigter C₁- bis C₉-Alkylrest oder Alkoxycarbonylrest ist.

Bevorzugt sind also Phenol, Alkylphenole wie Kresole, p-tert.-Butylphenol, p-Cumylphenol, p-n-Octylphenol, p-iso-Octylphenol, p-n-Nonylphenol und p-iso-Nonylphenol, Halogenphenole wie p-Chlorphenol, 2,4-Dichlorphenol, p-Bromphenol und 2,4,6-Tribromphenol oder Salicylsäuremethylester. Besonders bevorzugt ist Phenol.

Das eingesetzte Alkali zur Bildung des Phenolats kann eine Alkalilauge mit Hydroxiden aus der Reihe: Na-, K-, Li-Hydroxid sein, bevorzugt ist Natronlauge, und wird im neuen Verfahren vorzugsweise als 10 bis 55 Gew.-%-ige Lösung eingesetzt.

Die Reaktion b) kann durch Katalysatoren, wie tertiäre Amine, N-Alkylpiperidine oder Oniumsalze beschleunigt werden. Bevorzugt werden Tributylamin, Triethylamin und N-Ethylpiperidin verwendet.

Der eingesetzte Amin-Katalysator kann offenkettig oder cyclisch sein, besonders bevorzugt ist Triethylamin und Ethylpiperidin. Der Katalysator wird im neuen Verfahren vorzugsweise als 1 bis 55 Gew.%-ige Lösung eingesetzt.

Unter Oniumsalzen werden hier Verbindungen wie NR₄X verstanden, wobei R ein Alkyl und/oder Arylrest und/oder ein H sein kann und X ein Anion ist.

Phosgen kann in dem Verfahrensschritt b) flüssig, gasförmig oder gelöst in einem inerten Lösungsmittel eingesetzt werden.

In dem neuen Verfahren in Schritt b) bevorzugt verwendbare inerte organische Lösungsmittel sind beispielsweise Dichlormethan, Toluol, die verschiedenen Dichlorethane und Chlorpropanverbindungen, Chlorbenzol und Chlortoluol. Vorzugsweise wird Dichlormethan eingesetzt.

Die Reaktionsführung für Schritt b) erfolgt bevorzugt kontinuierlich und besonders bevorzugt in einer Pfropfenströmung ohne große Rückvermischung. Dies kann somit beispielsweise in Rohrreaktoren geschehen. Die Durchmischung der zwei Phasen (wässrige und organische Phase) kann durch eingebaute Rohrblenden, statische Mischer und/oder beispielsweise Pumpen realisiert werden.

Die Reaktion gemäß Schritt b) erfolgt besonders bevorzugt zweistufig.

In der ersten Stufe des bevorzugten Verfahrens wird die Reaktion durch Zusammenbringen der Edukte Phosgen, des inerten Lösungsmittels, welches bevorzugt erst als Lösungsmittel für das Phosgen dient, und des Monophenols, welches vorzugsweise zuvor bereits in der Alkalilauge gelöst ist, gestartet. Die Verweilzeit liegt typischerweise in der ersten Stufe im Bereich von 2 Sekunden bis 300 Sekunden, besonders bevorzugt im Bereich von 4 Sekunden bis 200 Sekunden. Der pH-Wert der ersten Stufe wird durch das Verhältnis von Alkalilauge/ Monophenol /Phosgen bevorzugt so eingestellt, dass der pH-Wert im Bereich von 11,0 bis 12,0, bevorzugt 11,2 bis 11,8, besonders bevorzugt bei 11,4 bis 11,6 liegt. Die Reaktionstemperatur der ersten Stufe wird durch Kühlung bevorzugt <40°C, besonders bevorzugt <35°C gehalten.

In der zweiten Stufe des bevorzugten Verfahrens wird die Reaktion zum Diarylcarbonat komplettiert. Die Verweilzeit beträgt beim bevorzugten Prozess 1 Minute bis 2 Stunden, bevorzugt 2 Minuten bis 1 Stunde, ganz besonders bevorzugt 3 Minuten bis 30 Minuten. In der zweiten Stufe des bevorzugten Verfahrens wird durch permanente Kontrolle des pH-Werts (wird im kontinuierlichen Verfahren bevorzugt Online nach grundsätzlich bekannten Verfahren gemessen) und entsprechende Einstellung des pH-Wertes durch Zugabe der Alkalilauge geregelt. Die Menge zugeführten Alkalilauge wird insbesondere so eingestellt, dass der pH-Wert der Reaktionsmischung in der zweiten Verfahrensstufe im Bereich von 7,5 bis 10,5, bevorzugt 8 bis 9,5, ganz besonders bevorzugt 8,2 bis 9,3 liegt. Die Reaktionstemperatur der zweiten Stufe wird durch Kühlung bei bevorzugt <50°C, besonders bevorzugt <40°C, ganz besonders bevorzugt <35°C gehalten.

Die in dieser Anmeldung aufgeführten allgemeinen oder in Vorzugsbereichen aufgeführten Parameter bzw. Erläuterungen können jedoch auch untereinander, also zwischen den jeweiligen Bereichen und Vorzugsbereichen beliebig kombiniert werden.

Im bevorzugten Verfahren wird in Schritt b) Phosgen in Bezug auf das Monophenol im Mol-Verhältnis von 1 2 bis 1 : 2,2 eingesetzt. Das Lösungsmittel wird so zugemischt, dass der Diarylcarbonat in einer 5 bis 60 %igen Lösung, bevorzugt 20 bis 45 %igen Lösung nach der Reaktion vorliegt.

Die Konzentration des Katalysators beträgt bevorzugt 0,0001 mol bis 0,1 mol, bezogen auf das eingesetzte Monophenol.

Nach der Reaktion b) wird in Schritt c) bevorzugt die organische, den Diarylcarbonat enthaltende Phase üblicherweise mit einer wässrigen Flüssigkeit gewaschen und nach jedem Waschvorgang von der wässrigen Phase soweit wie möglich getrennt. Die Wäsche erfolgt bevorzugt mit entsalztem Wasser. Die Diarylcarbonatlösung ist nach der Wäsche und Abtrennung der Waschflüssigkeit üblicherweise trüb. Als Waschflüssigkeit werden wässrige Flüssigkeiten zur Abtrennung des Katalysators, z.B. eine verdünnte Mineralsäure wie HCl oder H₃PO₄, und zur weiteren Reinigung vollentsalztes Wasser eingesetzt. Die Konzentration von HCl oder H₃PO₄ in der Waschflüssigkeit kann beispielsweise 0,5 bis 1,0 Gew.-% betragen. Die organische Phase wird beispielhaft und vorzugsweise zweimal gewaschen.

Als Phasentrennvorrichtungen zur Abtrennung der Waschflüssigkeit von der organischen Phase können grundsätzlich bekannte Trenngefäße, Phasenseparatoren, Zentrifugen oder Coalescer oder auch Kombinationen dieser Einrichtungen verwendet werden.

Man erhält so ohne Berücksichtigung des noch abzutrennenden Lösungsmittels überraschend hohe Reinheitsgrade des Diarylcarbonats von >99,85 %.

In einer bevorzugten Ausführungsform des erfindungsgemäßen Verfahrens werden die in Schritt c) abgetrennten Waschflüssigkeiten gegebenenfalls nach Trennung von Katalysatorresten und/oder organischen Lösungsmittelresten wieder in die Reaktion b) des erfindungsgemäßen Verfahrens zurückgeführt.

Dabei umfasst die Abtrennung und Aufarbeitung des in Schritt b) gebildeten Diarylcarbonats gemäß Schritt c) bevorzugt wenigstens die folgenden Schritte:
aa) Trennung von Diarylcarbonat-enthaltender organischer Phase und wässriger Alkalichlorid-haltiger Reaktionsabwasserlösung
bb) wenigstens einmaliges, bevorzugt wenigstens zweimaliges, besonders bevorzugt zweimaliges Waschen der in Schritt aa) erhaltenen Diarylcarbonat-enthaltenden organischen Phase und Abtrennen der jeweiligen Waschflüssigkeit.

Gegebenenfalls kann es erforderlich sein, wenigstens eine der gemäß Schritt c)bb) erhaltenen Waschflüssigkeit(en) von Katalysatorresten und gegebenenfalls organischen Lösungsmittelresten durch Einstellung des pH Wertes auf mindestens 9, bevorzugt mindestens 10, besonders bevorzugt 10 bis 11, durch Zugabe wenigstens einer basischen Verbindung zu trennen und einer anschließenden Extraktion mit wenigstens einem inerten organischen Lösungsmittel oder bevorzugt einem anschließenden Strippen der Lösung mit Wasserdampf zu unterziehen. Als basische Verbindungen zur Einstellung des pH-Wertes eignen sich beispielsweise Alkali- oder Erdalkalihydroxide oder -carbonate. Die basischen Verbindungen können in fester Form oder in Form ihrer wässrigen Lösungen eingesetzt werden. Bevorzugt werden Alkalilaugen, besonders bevorzugt Natronlauge verwendet.

Vorzugsweise wird wenigstens ein Teil der Waschflüssigkeit(en) aus Schritt c)bb) als Teilersatz des Wassers zur Herstellung der Natronlauge für Schritt b), insbesondere zur Einstellung der Konzentration der Natronlauge für Schritt b) verwendet. In einer bevorzugten Ausführungsform des erfindungsgemäßen Verfahrens wird wenigstens ein Teil der Waschflüssigkeit(en) aus Schritt c)bb) zur Verdünnung der gemäß Schritt e) hergestellten Alkalilauge verwendet, bevor diese in die Herstellung von Diarylcarbonat gemäß Schritt b) zurückgeführt wird. Die bevorzugte Ausführungsform des erfindungsgemäßen Verfahrens, bei der die in Schritt c) abgetrennten Waschflüssigkeiten wieder in das erfindungsgemäße Verfahren zurückgeführt werden bietet den zusätzlichen Vorteil geringerer Abwasseremission.

Nach der Synthese des Diarylcarbonats wird das Diarylcarbonat in Form seiner Lösung in dem bei der Synthese verwendeten organischen Lösungsmittel, beispielsweise Methylenchlorid, abgetrennt.

Zum Erhalt des hochreinen Diarylcarbonats wird das Lösungsmittel verdampft. Das Verdampfen kann in mehreren Verdampferstufen erfolgen. Beispielsweise erfolgt dies durch eine oder mehrere hintereinander geschaltete Destillationskolonnen bei der das Lösungsmittel vom Diarylcarbonat abgetrennt wird.

Diese Reinigungsstufe c) bzw. Stufen können beispielsweise kontinuierlich so geführt werden, dass die Sumpftemperatur bei der Destillation 150°C bis 310°C, bevorzugt 160 bis 230°C beträgt. Der zur Durchführung dieser Destillation angewendete Druck beträgt dabei insbesondere 1 bis 1000 mbar, bevorzugt 5 bis 100 mbar.

Die so gereinigten Diarylcarbonate zeichnen sich durch besonders hohe Reinheit (GC >99,95%) und extrem gutes Umesterungsverhalten aus, so dass hieraus anschließend ein Polycarbonat in exzellenter Qualität hergestellt werden kann.

Die Verwendung der Diarylcarbonate zur Herstellung von aromatischen Oligo-/Polycarbonaten nach dem Schmelzumesterungsverfahren ist literaturbekannt und beispielsweise in der Encyclopedia of Polymer Science, Vol. 10 (1969), Chemistry and Physics of Polycarbonates, Polymer Reviews, H. Schnell, Vol. 9, John Wiley and Sons, Inc. (1964) oder der US-A- 5 340 905 beschrieben.

Die verbleibende wässrige Alkalichlorid-haltige Reaktionsabwasserlösung gemäß Schritt c) wird vorteilhafterweise von leicht flüchtigen organischen Verunreinigungen, wie z.B. Reste des bei der Synthese verwendeten organischen Lösungsmittels und ggf. Katalysator beispielsweise durch Destillation oder Wasserdampfstrippen befreit. Es verbleibt dann ein Abwasser mit einem hohen Gehalt an gelöstem Natriumchlorid (10-20 Gew.-%) und gelösten Natriumcarbonaten (0,3-1,5 Gew.-%). Die Carbonate entstehen dabei z.B. durch Hydrolyse des Phosgens als Nebenreaktion der Diarylcarbonatherstellung. Außerdem ist das Abwasser mit organischen Verbindungen belastet, z.B. mit Phenolen (z.B. unsubstituiertes Phenol, Alkylphenole).

Die Behandlung des vorgereinigten Abwassers mit Adsorbentien erfolgt dann bevorzugt mit Aktivkohle.

Gemäß einem bevorzugten Verfahren wird die Erniedrigung des pH-Wertes im Prozessschritt d) mit Salzsäure oder Chlorwasserstoff durchgeführt. Der grundsätzlich denkbare, aber im vorliegenden Verfahren unerwünschte Einsatz der preiswerteren Schwefelsäure würde dazu führen, dass bei der pH-Erniedrigung Natriumsulfat entstünde, dass bei der anschließenden Elektrolyse im Anolytkreislauf angereichert werden würde. Da z.B. die Ionenaustauschermembranen gemäß Herstellerangaben nur bis zu einer bestimmten Natriumsulfat-Konzentration im Anolyten betrieben werden dürfen, müsste mehr Anolyt ausgeschleust werden als bei Einsatz von Salzsäure oder Chlorwasserstoff, dessen Reaktionsprodukt das gewünschte Natriumchlorid ist.

Das Verfahren der Alkalichlorid-Elektrolyse wird im folgenden näher beschrieben. Die nachstehende Beschreibung ist in Bezug auf die Elektrolyse von Natriumchlorid beispielhaft anzusehen, da im Verfahren wie bereits oben ausgeführt wurde grundsätzlich jedes Alkalichlorid zum Einsatz kommen kann (insbesondere Li-, Na-, KCl), die Verwendung von Natriumchlorid bzw. Natronlauge in den vorgehenden Stufen aber die bevorzugte Ausführung des Verfahrens ist.

Üblicherweise werden z.B. zur Elektrolyse von Natriumchlorid-haltigen Lösungen Membranelektrolyseverfahren eingesetzt (siehe hierzu Peter Schmittinger, CHLORINE, Wiley-VCH Verlag, 2000). Hierbei wird eine zweigeteilte Elektrolysezelle eingesetzt, die aus einem Anodenraum mit einer Anode und einem Kathodenraum mit einer Kathode besteht. Anoden- und Kathodenraum werden von einer Ionenaustauschermembran getrennt. In den Anodenraum wird eine Natriumchlorid-haltige Lösung mit einer Natriumchlorid-Konzentration von üblicherweise mehr als 300 g/l eingeführt. An der Anode wird das Chlorid-Ion zu Chlor oxidiert, dass mit der abgereicherten Natriumchlorid-haltigen Lösung (ca. 200 g/l) aus der Zelle geführt wird. Die Natrium-Ionen wandern unter Einfluss des elektrischen Feldes durch die Ionenaustauschermembran in den Kathodenraum. Bei dieser Wanderung nimmt jedes mol Natrium je nach Membran zwischen 3,5 und 4,5 mol Wasser mit. Dies führt dazu, dass der Anolyt an Wasser verarmt. Im Gegensatz zum Anolyten wird auf der Kathodenseite durch die Elektrolyse von Wasser zu Hydroxid-Ionen und Wasserstoff Wasser verbraucht. Das mit den Natrium-Ionen in den Katholyten gelangende Wasser reicht aus, um die Natronlauge-Konzentration im Auslauf auf 31-32 Gew.-% zu halten, dies bei einer Einlaufkonzentration von 30% und einer Stromdichte von 4 kA/m². Im Kathodenraum wird Wasser elektrochemisch reduziert, wobei Hydroxid-Ionen und Wasserstoff entstehen.

Alternativ kann als Kathode auch eine Gasdiffusionselektrode eingesetzt werden, an der Sauerstoff mit Elektronen zu Hydroxid-Ionen umgesetzt wird, wobei kein Wasserstoff entsteht. Mit den über die Ionenaustauschermembran in den Kathodenraum gelangten Natrium-Ionen bilden die Hydroxid-Ionen Natronlauge. In die Kathodenkammer wird üblicherweise eine Natronlauge mit einer Konzentration von 30 Gew.-% zugeführt und eine Natronlauge mit einer Konzentration von 31-32 Gew.-% abgeführt. Ziel ist es eine möglichst hohe Konzentration an Natronlauge zu erreichen, da üblicherweise die Natronlauge als 50 Gew.-%ige Lauge gelagert oder transportiert wird. Handelsübliche Membranen sind jedoch derzeit nicht beständig gegen eine Lauge mit einer höheren Konzentration als 32 Gew.-%, so dass die Natronlauge durch thermische Eindampfung aufkonzentriert werden muss.

Im Falle der Natriumchlorid-Elektrolyse wird über diese Natriumchlorid-haltige Lösung zusätzliches Wasser in den Anolyten eingebracht, jedoch nur Wasser über die Membran in den Katholyten ausgetragen. Wird mehr Wasser über die Natriumchlorid-haltige Lösung eingebracht als zum Katholyten transportiert werden kann, verarmt der Anolyt an Natriumchlorid und die Elektrolyse kann nicht kontinuierlich betrieben werden. Bei sehr geringen Natriumchlorid-Konzentrationen würde die Nebenreaktion der Sauerstoffbildung einsetzen.

Um maximale Mengen an Natriumchlorid-haltigen Lösungen wirtschaftlich der Natriumchlorid-Elektrolyse zuzuführen, kann es nützlich sein, dass der Wassertransport über die Membran erhöht wird. Dieser kann durch Auswahl geeigneter Membranen erfolgen, wie in der US-A-4025405 beschrieben. Der Effekt eines erhöhten Wassertransportes ist, dass auf die sonst übliche Wasserzugabe zur Aufrechterhaltung der Laugekonzentration verzichtet werden kann.

Gemäß der US-A-3 773 634 kann bei hohem Wassertransport durch die Membran die Elektrolyse dann betrieben werden, wenn eine Laugekonzentration von 31 bis 43 Gew.-% und eine Natriumchlorid-Konzentration von 120 bis 250 g/l eingesetzt wird.

Nachteil beider Verfahren ist die niedrigere Stromausbeute dieser Verfahren.

Gemäß dem bevorzugten Verfahren erfolgt die Abtrennung d) des Natriumchlorid-haltigen Reaktionsabwassers nach der Phasentrennung und der Entfernung des Lösungsmittels und gegebenenfalls verwendeten Katalysators, durch Strippung mit Wasserdampf und, nach der pH-Einstellung, durch eine Aktivkohlebehandlung.

Hiernach kann das Alkalichlorid-haltige Abwasser direkt der Elektrolyse e) zugeführt werden.

Gegenüber dem Stand der Technik (WO 03/70639), in dem max. 26 % des im Abwasser der DPC-Produktion vorhandenen Natriumchlorids bei der NaCl-EIektrolyse eingesetzt werden kann, können durch das erfindungsgemäße Verfahren mehr als 26 % des Natriumchlorids aus dem Abwasser zurück gewonnen werden.

Eine weitere bevorzugte Verfahrensvariante ist, dass dem Alkalichlorid-haltigen Abwasser durch ein Aufkonzentrierungsverfahren Wasser entzogen wird. Bevorzugt ist daher ein Verfahren, dadurch gekennzeichnet, dass die Alkalichlorid-haltige Lösung aus d) vor der Elektrolyse e) mittels Membrandestillationsverfahren oder Umkehrosmose aufkonzentriert wird.

Hierbei können beispielsweise die Umkehrosmose oder besonders bevorzugt die Membrandestillation oder Membrankontaktoren eingesetzt werden (siehe MELIN; RAUTENBACH, Membranverfahren; SPRINGER, BERLIN, 2003). Durch Kombination von erfindungsgemäßem Betrieb der Elektrolysezellen und Aufkonzentrierungsverfahren können theoretisch bis zu 100 % des Natriumchlorids aus dem Abwasser wieder gewonnen werden.

Das neue Verfahren kann auch mit einer Alkalichlorid-Elektrolyse durchgeführt werden, bei der kein Wasserstoff an der Kathode erzeugt wird, sondern die Kathode durch eine Gasdiffusionselektrode ersetzt wird, an der Sauerstoff zu Hydroxid-Ionen reduziert wird.

Wenn z.B. in einem Verbundstandort kein Wasserstoff für chemische Reaktionen benötigt wird, kann auf das Zwangsanfallprodukt Wasserstoff verzichtet werden. Vorteil ist eine Energieeinsparung bei der Elektrolyse, die durch die niedrigere Elektrolysespannung bei Einsatz einer Gasdiffusionselektrode zurückzuführen ist.

Die aus der DPC-Produktion gelangende Natriumchlorid-haltige Lösung weist üblicherweise einen Natriumchlorid-Gehalt von bis zu 17 Gew.-% auf, soweit es sich um das Reaktionsabwasser handelt. Wird das Reaktionsabwasser mit dem Waschwasser vereinigt so beträgt die NaCl-Konzentration beispielsweise ca. 13 Gew.%. Liefert die Elektrolyse das Chlor und die Natronlauge ausschließlich für die DPC-Produktion, so kann das Natriumchlorid-haltige Abwasser nur zu einem geringen Teil in der Elektrolyse eingesetzt werden. So können bei den üblichen Ionenaustauschermembranen und den Standardbetriebsparametern der Natriumchlorid-Elektrolyse nur max. 26 % des Natriumchlorids eines 17 Gew.-%igen Natriumchlorid-haltigen DPC-Abwassers eingesetzt werden. Die Standardbetriebsparameter der NaCl-Elektrolyse sind eine Solekonzentration im Ablauf von 200 bis 240g/l und eine NaOH-Konzentration von 31-32 Gew.-%. Ein vollständiges Recycling des anfallenden Natriumchlorids ist daher bisher nicht möglich. Eine Aufkonzentrierung durch thermische Verdampfung des Wassers ist derzeit nicht wirtschaftlich, da das Natriumchlorid als sehr preiswertes Produkt zur Verfügung steht.

Mit dem erfindungsgemäßen Verfahren können deutlich mehr als 26 % des Natriumchlorid aus anfallenden Abwässern, bei einer Konzentration von 17 Gew.-% recycelt werden, sofern die Natriumchlorid-Elektrolyse ausschließlich das Chlor und die Natronlauge für die DPC-Produktion bereitstellt. Üblicherweise werden Natriumchlorid-Elektrolysen an chemischen Verbundstandorten mit mehreren Chlorverbrauchern betrieben, so dass nicht von allen Verbrauchern eine Natriumchlorid-haltige Lösung zum Recycling bereit steht. Der Anteil an wieder verwertbarem Natriumchlorid aus dem Abwasser steigt, wenn die Natriumchlorid-Elektrolyse die Natronlauge und das Chlor nicht ausschließlich für die Diarylcarbonat-Produktion bereitstellen muss.

Eine weitere bevorzugte Variante des neuen Verfahrens ist, dass das Abwasser der Diarylcarbonatherstellung durch festes Alkalichlorid aufkonzentriert und der Alkalichlorid-Elektrolyse zugeführt wird. Hierdurch könnten mehr als 50% des Alkalichlorids aus dem DPC-Abwasser wieder verwendet werden.

Dies setzt jedoch voraus, dass das Chlor und die Alkalilauge nicht ausschließlich für die Diarylcarbonat-Produktion eingesetzt wird.

Besonders bevorzugt wird ein Alkalichlorid-haltiges Abwasser bei der Elektrolyse e) eingesetzt bzw. zugeführt dessen pH-Wert kleiner als 7 beträgt. Die pH-Wert Einstellung erfolgt vorzugsweise mit Salzsäure, kann aber auch mit gasförmigem Chlorwasserstoff erfolgen.

Gemäß einem weiteren bevorzugten Verfahren wird die NaCl-Elektrolyse so betrieben, dass die NaCl-Lösung, die aus der Zelle gelangt, eine NaCl-Konzentration von weniger als 200 g/l aufweist. Parallel hierzu kann die aus der Zelle ablaufende Laugenkonzentration weniger als 30 Gew.-% betragen.

Der Wassertransport über die Membran hängt nicht nur von den Betriebsparametern ab, sondern auch von dem eingesetzten Membran-Typ. Gemäß dem erfindungsgemäßen Verfahren werden bevorzugt solche Ionenaustauschermembranen eingesetzt, die unter den erfindungsgemäßen Bedingungen der Natriumchlorid- und Laugekonzentration einen Wassertransport durch die Membran von mehr als 4,5 mol Wasser je mol Natrium ermöglichen.

Die Stromdichte wird dabei auf die Membranfläche berechnet und beträgt insbesondere 2 bis 6 kA/m². Besonders bevorzugt werden Anoden mit größere Oberfläche eingesetzt. Unter Anoden mit größerer Oberfläche sind solche zu verstehen, bei denen die physikalische Oberfläche deutlich höher ist als die projekzierte Oberfläche. Anoden mit größerer Oberfläche sind z.B. Schaum- oder Filz-artig aufgebaute Elektroden. Hierdurch wird anodische eine sehr große Elektrodenoberfläche angeboten und die lokale Stromdichte stark erniedrigt. Die Oberfläche der Anode ist bevorzugt so zu wählen, das die lokale Stromdichte bezogen auf die physikalische Oberfläche der Elektrode kleiner als 3 kA/m² beträgt. Je größer die Oberfläche und je niedriger die lokale Stromdichte, desto gering kann die Natriumchlorid-Konzentration in der Sole gewählt werden und desto höher ist der Anteil an Natriumchlorid aus dem Abwasser, der recycelt werden kann.

Der pH-Wert des Alkalichlorid-haltigen Abwassers sollte vor der Elektrolyse e) bevorzugt kleiner als 7 betragen, besonders bevorzugt von 0,5 bis 6.

Die Alkalichlorid-Elektrolyse sollte so betrieben werden, dass die Alkalichlorid-Konzentration der aus der Zelle gelangenden Alkalichlorid-Lösung zwischen 100 bis 280 g/l Natriumchlorid und/oder dass die Konzentration der Alkalilauge, die aus der Zelle gelangt 13 bis 33 Gew.-% beträgt.

Besonders bevorzugt sind Konzentrationen, die den Betrieb der Zelle bei niedrigeren Spannungen ermöglichen. Hierzu sollte die Konzentration der aus der Zelle gelangenden Alkalichlorid-Lösung vorzugsweise zwischen 110 bis 220 g/l Alkalichlorid und/oder die Konzentration der Alkalilauge, die aus der Zelle gelangt 20 bis 30 Gew.-% betragen.

Die eingesetzten Ionenaustauschermembranen bei der Elektrolyse sollten bevorzugt einen Wassertransport je mol Natrium von mehr als 4,0 mol H₂O/mol Natrium aufweisen, besonders bevorzugt 5,5 bis 6,5 mol H₂O/mol Natrium.

Das Verfahren wird bevorzugt so betrieben, dass die Elektrolyse e) bei einer Temperatur von 70 bis 100°C, vorzugsweise bei 80 bis 95°C betrieben wird.

Die Elektrolyse wird bei einem Absolutdruck von 1 bis 1,4 bar, bevorzugt bei einem Druck von 1,1 bis 1,2 bar betrieben.

Die Druckverhältnisse zwischen Anoden- und Kathodenraum werden insbesondere so gewählt, dass der Druck im Kathodenraum höher ist als der Druck im Anodenraum.

Der Differenzdruck zwischen Kathoden- und Anodenraum sollte in einem besonders bevorzugten Verfahren 20 bis 150 mbar, vorzugsweise 30 bis 100 mbar betragen.

Bei niedrigeren Alkalichlorid-Konzentrationen können auch spezielle Anodencoatings eingesetzt werden. Insbesondere kann das Coating der Anode neben Rutheniumoxid auch weitere Edelmetallkomponenten der 7. und 8. Nebengruppe des Periodensystems der Elemente enthalten. Beispielsweise kann das Anodencoating mit Palladiumverbindungen dotiert werden. Ebenso sind Coatings auf Basis von Diamanten einsetzbar.

Die nachfolgenden Beispiele sollen die vorliegende Erfindung illustrieren ohne sie jedoch einzuschränken.

### Beispiele

Die Beispiele sollen das erfindungsgemäße Verfahren anhand des bei der Herstellung von Diphenylcarbonat anfallenden Natriumchlorid-haltigen Abwassers darstellen.

### Beispiel 1

Zusatz von Natriumchlorid-haltigem Reaktionsabwasser in die Natriumchlorid-Elektrolyse - Zugabe einer 17 Gew.%igen Natriumchlorid-Lösung aus der DPC-Herstellung

In einen senkrecht stehenden, gekühlten Rohrreaktor wird kontinuierlich ein Gemisch aus 145,2 kg/h 14,5 Gew.-%iger Natronlauge und 48,3 kg/h Phenol mit einer Lösung von 86,2 kg/h Methylenchlorid und 27,5 kg/h Phosgen (8 Mol-% Überschuss bzgl. auf Phenol) zusammengebracht. Dieses Reaktionsgemisch wird auf eine Temperatur von 33°C gekühlt und nach einer mittleren Verweilzeit von 15 Sekunden ein pH-Wert von 11,5 gemessen. Zu diesem Reaktionsgemisch werden nun in der zweiten Stufe des Verfahrens 5,4 kg/h 50%iger NaOH zudosiert, so dass der pH-Wert der zweiten Reaktionsstufe nach einer weiteren Verweilzeit von 5 Minuten 8,5 beträgt. In der kontinuierlich betriebenen Reaktion werden auftretende Dosierschwankungen durch jeweilige Anpassungen der NaOH-Dosierungen aufgefangen. In der zweiten Stufe des Verfahrens wird das Reaktionsgemisch durch das Durchleiten eines mit Verengungen versehen Rohres ständig gemischt. Die Reaktionstemperatur wird nach erneuter Zugabe der NaOH durch Kühlen auf 30°C eingestellt. Nach Abtrennung der organischen von der wässrigen Phase (Reaktionsabwasser) wird die DPC-Lösung mit 0,6 %-iger Salzsäure und Wasser gewaschen. Nach Entfernung des Lösungsmittels wird 99,9 %-iges Diphenylcarbonat erhalten. Das Reaktionsabwasser wird hier nicht mit den Waschphasen vereinigt und wird durch Strippen mit Wasserdampf von Lösungsmittelresten und Katalysator befreit. Nach Neutralisierung mit Salzsäure und Behandlung mit Aktivkohle enthält das Reaktionsabwasser 17% NaCl und < 2 ppm Phenol.

Es kann ohne weitere Reinigung der Natriumchlorid-Elektrolysezelle zugeführt werden.

Die Elektrolyse wird beispielhaft in einer Laborelektrolysezelle mit einer Anodenfläche von 0,01m² durchgeführt. Die Stromdichte betrugt 4 kA/m², Temperatur Auslauf Kathodenseite 88°C, Temperatur Auslauf Anodenseite 89°C. Eingesetzt wurde eine Elektrolysezelle mit Standard Anoden- und Kathodencoating der Fa. DENORA, Deutschland. Eingesetzt wurde eine Ionenaustauschermembran der Fa. DuPont Nafion 982 WX. Die Elektrolysespannung betrug 3,02 V. Durch die Anodenkammer wurde eine Natriumchlorid-haltige Lösung mit einem Massenstrom von 0,98 kg/h umgepumpt. Die Konzentration der der Anodenkammer zugeführten Lösung betrug 25 Gew.-% NaCl. Aus der Anodenkammer konnte eine 20 Gew.-%ige NaCl-Lösung entnommen werden. Der aus der Anodenkammer entnommenen NaCl-Lösung wurden 0,121 kg/h an 17 Gew.-%igen Reaktionsabwasser aus der Diphenylcarbonatherstellung und 0,0653 kg/h festes Natriumchlorid zugegeben. Die Lösung wurde anschließend wieder in die Anodenkammer eingespeist. Der Wassertransport über die Membran betrug 3,5 mol Wasser je mol Natrium.

Auf der Kathodenseite wurde eine Natronlauge mit einem Massenstrom von 1,107 kg/h umgepumpt. Die Konzentration der in die Kathodenseite eingespeisten Natronlauge betrug 30 Gew.-% NaOH, die aus der Kathodenseite entnommene Natronlauge hatte eine Konzentration von 32 % NaOH. 0,188 kg/h der 31,9%igen Lauge wurden dem Volumenstrom entnommen, der Rest wurde mit 0,0664 kg/h Wasser aufgestockt und wieder in das Kathodenelement zurückgeführt.

23,3 % des umgesetzten Natriumchlorids stammen aus dem DPC-Reaktionsabwasser.

### Beispiel 2

Zusatz von Natriumchlorid-haltigem Reaktionsabwasser in die Natriumchlorid-Elektrolyse mit Gasdiffusionselektrode - Zugabe einer 17 Gew.-%igen Natriumchlorid-Lösung (Reaktionsabwasser) aus der DPC-Herstellung.

Das Abwasser entsprach der Qualität des gemäß Beispiel 1. Da für die Herstellung von DPC kein Wasserstoff benötigt wird, kann auf die Bildung von Wasserstoff bei der Elektrolyse verzichtet werden. Die Elektrolyse wurde daher mit Gasdiffusionselektroden betrieben. Die Stromdichte betrugt 4 kA/m², Temperatur Auslauf Kathodenseite 88°C, Temperatur Auslauf Anodenseite 89 °C. Eingesetzt wurde eine Elektrolysezelle mit Standard Anoden-Coating der Fa. DENORA, Deutschland. Eingesetzt wurde eine Ionenaustauschermembran der Fa.DuPont, Nafion 982 WX. Die Elektrolysespannung betrug 2,11 V. Die Natriumchlorid-Konzentration der der Anodenkammer entnommenen Lösung betrug 17 Gew.% an NaCl. Der aus der Anodenkammer entnommenen NaCl-Lösung wurden 0,166 kg/h an 17 Gew.-%igem Reaktionsabwasser und 0,0553 kg/h festes Natriumchlorid zugegeben. Die Lösung wurde anschließend wieder in die Anodenkammer eingespeist. Der Wassertransport über die Membran betrug 4,9 mol Wasser je mol Natrium.

Auf der Kathodenseite wurde eine Natronlauge mit einem Massenstrom von 0,848 kg/h umgepumpt. Die Konzentration der in die Kathodenseite eingespeisten Natronlauge betrug 30 Gew.-% NaOH, die aus der Kathodenseite entnommen Natronlauge hatte eine Konzentration von 32 Gew.% NaOH. 0,192 kg/h der 31,2%igen Lauge wurden dem Volumenstrom entnommen, der Rest wurde mit 0,033 kg/h Wasser aufgestockt und wieder in das Kathodenelement zurückgeführt.

Der Anteil an umgesetztem Natriumchlorid aus dem DPC-Reaktionsabwasser betrug 32,4%.

### Beispiel 3

Zusatz von Natriumchlorid-haltigem Reaktionsabwasser in die Natriumchlorid-Elektrolyse mit Gasdiffusionselektrode - Zugabe einer 17 Gew.-%igen Natriumchlorid-Lösung (Reaktionsabwasser) aus der DPC-Herstellung.

Das Abwasser entsprach der Qualität des gemäß Beispiel 1. Da für die Herstellung von DPC kein Wasserstoff benötigt wird, kann auf die Bildung von Wasserstoff bei der Elektrolyse verzichtet werden. Die Elektrolyse wurde daher mit Gasdiffusionselektroden betrieben. Die Stromdichte betrugt 4 kA/m², Temperatur Auslauf Kathodenseite 88°C, Temperatur Auslauf Anodenseite 89°C. Eingesetzt wurde eine Elektrolysezelle mit Standard Anoden-Coating der Fa. DENORA, Deutschland. Eingesetzt wurde eine Ionenaustauschermembran der Fa.DuPont, Nafion 2030. Die Elektrolysespannung betrug 1,96 V. Die Natriumchlorid-Konzentration der der Anodenkammer entnommenen Lösung betrug 15 Gew.-% an NaCl. Der aus der Anodenkammer entnommenen NaCI-Lösung wurden 0,178 kg/h an 17 Gew.-%igem Reaktionsabwasser und 0,0553 kg/h festes Natriumchlorid zugegeben. Die Lösung wurde anschließend wieder in die Anodenkammer eingespeist. Der Wassertransport über die Membran betrug 5,26 mol Wasser je mol Natrium.

Auf der Kathodenseite wurde eine Natronlauge mit einem Massenstrom von 0,295 kg/h umgepumpt. Die Konzentration der in die Kathodenseite eingespeisten Natronlauge betrug 30 Gew.-% NaOH, die aus der Kathodenseite entnommen Natronlauge hatte eine Konzentration von 32 Gew.% NaOH. 0,188 kg/h der 32%igen Lauge wurden dem Volumenstrom entnommen, der Rest wurde mit 0,0184 kg/h Wasser aufgestockt und wieder in das Kathodenelement zurückgeführt.

Der Anteil an umgesetztem Natriumchlorid aus dem DPC-Reaktionsabwasser betrug 34,4 %.

### Beispiel 4

Rückführung von Waschphasen aus der DPC-Aufarbeitung in die DPC-Herstellung - Zugabe einer Abwasserphase aus der sauren Wäsche in die DPC-Herstellung.

Die Durchführung erfolgte wie in Beispiel 1, mit dem Unterschied, dass nach Abtrennung der organischen von der wässrigen Phase (Reaktionsabwasser) die DPC-Lösung mit 0,6 Gew.%-iger Salzsäure (saure Wäsche) und anschließend noch einmal mit Wasser (neutrale Wäsche) gewaschen wurde.

Die saure Waschphase aus der DPC-Aufarbeitung wurde mit NaOH auf pH 10 eingestellt und anschließend durch Extraktion mit Methylenchlorid oder durch Strippen mit Wasserdampf von Lösungsmittelresten und Katalysator befreit. Nach Phasentrennung erhielt man eine Wasserphase mit 1,5 Gew.-% NaCl, die als Teilersatz des Wassers zur Zubereitung der 14,5 Gew.-%-igen NaOH-Lösung für die DPC-Herstellung wiederverwendet werden konnte.

### Beispiel 5

Rückführung von Waschphasen aus der DPC-Aufarbeitung in die DPC-Herstellung - Zugabe einer neutralen Waschphase in die DPC-Herstellung.

Die Durchführung erfolgte wie in Beispiel 4.

Die neutrale Waschphase aus der DPC-Aufarbeitung konnte ohne weitere Behandlung als Teilersatz des Wassers zur Zubereitung der 14,5 Gew.-%-igen NaOH-Lösung für die DPC-Herstellung wiederverwendet werden.

### Beispiel 6

Rückführung von Waschphasen aus der DPC-Aufarbeitung in die DPC-Herstellung - Zugabe der vereinigten Abwasserphasen aus der sauren und neutralen Wäsche in die DPC-Herstellung

Die Durchführung erfolgte wie in Beispiel 4. Die saure und neutrale Waschphase aus der DPC-Aufarbeitung wurden vereinigt, mit NaOH auf pH 10 eingestellt und anschließend durch Extraktion mit Methylenchlorid oder durch Strippen mit Wasserdampf von Lösungsmittelresten und Katalysator befreit. Nach Phasentrennung erhielt man eine Wasserphase mit etwa 1 Gew.-% NaCl, die als Teilersatz des Wassers zur Zubereitung der 14,5 Gew.%-igen NaOH-Lösung für die DPC-Herstellung wiederverwendet werden konnte.

## Patentansprüche

1. Verfahren zur Herstellung von Diarylcarbonat und Verarbeitung mindestens eines Teils der dabei anfallenden Alkalichlorid-haltigen Lösung in einer nachgeschalteten Alkalichlorid-Elektrolyse, umfassend die folgenden Schritte:
a) Herstellung von Phosgen durch Umsetzung von Chlor mit Kohlenmonoxid,
b) Umsetzung des gemäß Schritt a) gebildeten Phosgens mit wenigstens einem Monophenol in Gegenwart einer Base und ggf. basischem Katalysator zu einem Diarylcarbonat und einer Alkalichlorid-haltigen Lösung,
c) Abtrennung und Aufarbeitung des in Schritt b) gebildeten Diarylcarbonats,
d) Abtrennung der gemäß Schritt c) verbliebenen Alkalichlorid-haltigen Lösung von Lösungsmittelresten und ggf. Katalysatorresten, insbesondere durch Strippen der Lösung mit Wasserdampf, und Behandlung mit Adsorbentien, insbesondere mit Aktivkohle,
e) Elektrochemische Oxidation wenigstens eines Teils der Alkalichlorid-haltigen Lösung aus d) unter Bildung von Chlor, Alkalilauge und ggf. Wasserstoff,
**dadurch gekennzeichnet, dass** bei der Abtrennung d) der Lösung vor der Behandlung mit Adsorbentien die Lösung auf einen pH-Wert kleiner oder gleich 8 eingestellt wird und
f) wenigstens ein Teil des gemäß Schritt e) hergestellten Chlors in die Herstellung von Phosgen gemäß Schritt a) zurückgeführt wird und/oder
g) wenigstens ein Teil des gemäß Schritt e) hergestellten Alkalilauge in die Herstellung von Diarylcarbonat gemäß Schritt b) zurückgeführt wird.

2. Verfahren gemäß Anspruch 1, **dadurch gekennzeichnet, dass** die elektrochemische Oxidation wenigstens eines Teiles der Alkalichlorid-haltigen Lösung aus d) zu Chlor und Natronlauge unter Einsatz von Gasdiffusionselektroden als Kathode erfolgt.

3. Verfahren gemäß Anspruch 1, **dadurch gekennzeichnet, dass** wenigstens ein Teil der gereinigten Alkalichlorid-haltigen Lösung aus d) in den Solekreislauf einer Membranelektrolyse zur Herstellung von Chlor, Natronlauge und ggf. Wasserstoff gegeben wird.

4. Verfahren gemäß einem der Ansprüche 1 bis 3, **dadurch gekennzeichnet, dass** der Alkalichlorid-haltigen Lösung bei der Elektrolyse e) zusätzliches Alkalichlorid zur Erhöhung der Alkalichlorid-Konzentration zugegeben wird.

5. Verfahren gemäß einem der Ansprüche 1 bis 4, **dadurch gekennzeichnet, dass** die Alkalichlorid-haltige Lösung bei der Reinigung d) auf einen pH-Wert von kleiner als 7 eingestellt ist, insbesondere durch Einsatz von Salzsäure oder Chlorwasserstoff.

6. Verfahren gemäß einem der Ansprüche 1 bis 5, **dadurch gekennzeichnet, dass** die Alkalichlorid-Konzentration, der in die Elektrolyse gelangenden Alkalichlorid-Lösung von 100 bis 280 g/l, bevorzugt 110 bis 220 g/l beträgt und/oder dass die Konzentration der Natronlauge, die aus der Elektrolyse erhalten wird, 13 bis 33 Gew.-%, bevorzugt 20 bis 32 Gew.-%, beträgt.

7. Verfahren gemäß einem der Ansprüche 1 bis 6, **dadurch gekennzeichnet, dass** Ionenaustauschermembranen bei der Elektrolyse e) eingesetzt werden, deren Wassertransport je mol Natrium größer ist als 4 mol H₂O/mol Natrium.

8. Verfahren gemäß Anspruch 7, **dadurch gekennzeichnet, dass** vorzugsweise Ionenaustauschermembranen bei der Elektrolyse e) eingesetzt werden, deren Wassertransport je mol Natrium 5,5 bis 6,5 mol H₂O/mol Natrium beträgt.

9. Verfahren gemäß einem der Ansprüche 1 bis 8, **dadurch gekennzeichnet, dass** die Elektrolyse bei einer Stromdichte von 2 bis 6 kA/m² betrieben wird, wobei die zugrundegelegte Fläche zur Berechnung der Stromdichte die Membranfläche ist.

10. Verfahren gemäß einem der Ansprüche 1 bis 9, **dadurch gekennzeichnet, dass** die Elektrolyse e) bei einer Temperatur von 70 bis 100°C, vorzugsweise bei 80 bis 95°C betrieben wird.

11. Verfahren gemäß einem der Ansprüche 1 bis 10, **dadurch gekennzeichnet, dass** die Elektrolyse bei einem Absolutdruck von 1,0 bis 1,4 bar, bevorzugt 1,1 bis 1,3 bar betrieben wird.

12. Verfahren gemäß einem der Ansprüche 1 bis 11, **dadurch gekennzeichnet, dass** die Elektrolyse e) mit einem Differenzdruck zwischen Kathoden- und Anodenraum von 20 bis 150 mbar, vorzugsweise 30 bis 100 mbar betrieben wird.

13. Verfahren gemäß einem der Ansprüche 1 bis 12, **dadurch gekennzeichnet, dass** die Elektrolyse mit einer Anode betrieben wird, die als Beschichtung neben Rutheniumoxid auch weitere Edelmetallverbindungen der 7. und 8. Nebengruppe und/oder der 4. Hauptgruppe des Periodensystems der Elemente enthält.

14. Verfahren gemäß einem der Ansprüche 1 bis 13, **dadurch gekennzeichnet, dass** in der Elektrolyse e) in den Zellen Anoden mit größerer Oberfläche als die Oberfläche der Membranen eingesetzt werden.

15. Verfahren gemäß einem der Ansprüche 1 bis 14, **dadurch gekennzeichnet, dass** die Alkalichlorid-haltige Lösung aus d) vor der Elektrolyse e) mittels Membranverfahren aufkonzentriert wird.

16. Verfahren gemäß einem der Ansprüche 1 bis 15, **dadurch gekennzeichnet, dass** als Monophenol in Stufe b) Phenol, Alkylphenole, insbesondere Kresole, p-tert.-Butylphenol, p-Cumylphenol, p-n-Octylphenol, p-iso-Octylphenol, p-n-Nonylphenol und p-isoNonylphenol, Halogenphenole insbesondere p-Chlorphenol, 2,4-Dichlorphenol, p-Bromphenol und 2,4,6-Tribromphenol, besonders bevorzugt Phenol eingesetzt wird.

17. Verfahren nach einem der Ansprüche 1 bis 16, **dadurch gekennzeichnet, dass** die Alkalichlorid-haltige Lösung des Reaktionsabwassers aus b) oder wenigstens teilweise mit Waschwasser aus der Aufarbeitung c) des Diarylcarbonats vereinigt eingesetzt wird.

18. Verfahren nach einem der Ansprüche 1 bis 17, **dadurch gekennzeichnet, dass** Phenole der Formel (I) 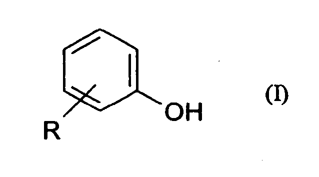 worin R Wasserstoff, Halogen oder ein verzweigter oder unverzweigter C₁ bis C₉-Alkylrest oder Alkoxycarbonylrest ist, in der Reaktion b) eingesetzt werden.

19. Verfahren nach einem der Ansprüche 1 bis 18, **dadurch gekennzeichnet, dass** Schritt c) wenigstens die folgenden Schritte umfasst:
aa) Trennung von Diarylcarbonat-enthaltender organischer Phase und wässriger Alkalichlorid-haltiger Reaktionsabwasserlösung
bb) wenigstens einmaliges Waschen der in Schritt aa) erhaltenen Diarylcarbonatenthaltenden organischen Phase,
und wobei wenigstens eines Teils der Waschphase(n) aus c)bb) gegebenenfalls nach Trennung von Katalysatorresten und gegebenenfalls organischen Lösungsmittelresten als Teilersatz des Wassers zur Herstellung der Natronlauge für Schritt b) verwendet wird.

## Claims

1. Process for producing diaryl carbonate and processing at least a part of the resultant alkali metal chloride-containing solution in a downstream alkali metal chloride electrolysis comprising the following steps:
a) production of phosgene by reacting chlorine with carbon monoxide,
b) reacting the phosgene formed according to step a) with at least one monophenol in the presence of a base and, if appropriate, basic catalyst, to give a diaryl carbonate and an alkali metal chloride-containing solution,
c) separating off and working up the diaryl carbonate formed in step b),
d) separating off the alkali metal chloride-containing solution remaining according to step c) from solvent residues and if appropriate catalyst residues, in particular by stripping the solution with steam, and treatment with adsorbents, in particular with activated carbon,
e) electrochemical oxidation of at least a part of the alkali metal chloride-containing solution from d), with formation of chlorine, alkali metal hydroxide solution and, if appropriate, hydrogen,
**characterized in that**, in the separation d) of the solution before treatment with adsorbents, the solution is adjusted to a pH less than or equal to 8 and
f) at least a part of the chlorine produced according to step e) is recirculated into the production of phosgene according to step a) and/or
g) at least a part of the alkali metal hydroxide solution produced according to step e) is recirculated into the production of diaryl carbonate according to step b).

2. Process according to Claim 1, **characterized in that** the electrochemical oxidation of at least a part of the alkali metal chloride-containing solution from d) to give chlorine and sodium hydroxide solution proceeds using gas diffusion electrodes as cathode.

3. Process according to Claim 1, **characterized in that** at least a part of the purified alkali metal chloride-containing solution from d) is added to the brine circuit of a membrane electrolysis for producing chlorine, sodium hydroxide solution and, if appropriate, hydrogen.

4. Process according to one of Claims 1 to 3, **characterized in that**, to increase the alkali metal chloride concentration, additional alkali metal chloride is added to the alkali metal chloride-containing solution in the electrolysis e).

5. Process according to one of Claims 1 to 4, **characterized in that** the alkali metal chloride-containing solution is adjusted, in the purification d), to a pH of less than 7, in particular by adding hydrochloric acid or hydrogen chloride.

6. Process according to one of Claims 1 to 5, **characterized in that** the alkali metal chloride concentration of the alkali metal chloride solution passing into the electrolysis is from 100 to 280 g/l, preferably 110 to 220 g/l, and/or **in that** the concentration of the sodium hydroxide solution which is obtained from the electrolysis is 13 to 33% by weight, preferably 20 to 32% by weight.

7. Process according to one of Claims 1 to 6, **characterized in that**, in the electrolysis e), use is made of ion-exchange membranes, the water transport of which, per mole of sodium, is greater than 4 mol of H₂O/mol of sodium.

8. Process according to Claim 7, **characterized in that**, in the electrolysis e), preferably use is made of ion-exchange membranes, the water transport of which, per mole of sodium, is 5.5 to 6.5 mol of H₂O/mol of sodium.

9. Process according to one of Claims 1 to 8, **characterized in that** the electrolysis is operated at a current density of 2 to 6 kA/m², with the surface area used as a basis for calculation of the current density being the membrane surface area.

10. Process according to one of Claims 1 to 9, **characterized in that** the electrolysis e) is operated at a temperature of 70 to 100°C, preferably 80 to 95°C.

11. Process according to one of Claims 1 to 10, **characterized in that** the electrolysis is operated at an absolute pressure of 1.0 to 1.4 bar, preferably 1.1 to 1.3 bar.

12. Process according to one of Claims 1 to 11, **characterized in that** the electrolysis e) is operated at a differential pressure between cathode space and anode space of 20 to 150 mbar, preferably 30 to 100 mbar.

13. Process according to one of Claims 1 to 12, **characterized in that** the electrolysis is operated using an anode which, as coating, in addition to ruthenium oxide, also contains other noble metal compounds of subgroups 7 and 8 and/or of main group 4 of the Periodic Table of the Elements.

14. Process according to one of Claims 1 to 13, **characterized in that**, in the electrolysis e), in the cells, use is made of anodes having a larger surface area than the surface area of the membranes.

15. Process according to one of Claims 1 to 14, **characterized in that** the alkali metal chloride-containing solution from d) is concentrated by means of membrane processes before the electrolysis e).

16. Process according to one of Claims 1 to 15, **characterized in that**, as monophenol in stage b), use is made of phenol, alkylphenols, in particular cresols, p-tert-butylphenol, p-cumylphenol, p-n-octylphenol, p-isooctylphenol, p-n-nonylphenol and p-isononylphenol, halophenols, in particular p-chlorophenol, 2,4-dichlorophenol, p-bromophenol and 2,4,6-tribromophenol, particularly preferably phenol.

17. Process according to one of Claims 1 to 16, **characterized in that** use is made of the alkali metal chloride-containing solution of the reaction wastewater from b), or at least in part combined with wash water from the workup c) of the diaryl carbonate.

18. Process according to one of Claims 1 to 17, **characterized in that** use is made of phenols of the formula (I) 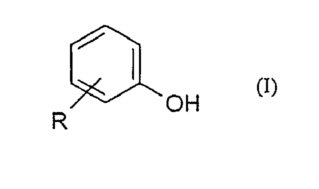 where R is hydrogen, halogen, or a branched or unbranched C₁ to C₉ alkyl radical or alkoxycarbonyl radical, in the reaction b).

19. Process according to one of Claims 1 to 18, **characterized in that** step c) comprises at least the following steps:
aa) separation of diaryl carbonate-containing organic phase and aqueous alkali metal chloride-containing reaction wastewater solution
bb) at least once washing the diaryl carbonate-containing organic phase obtained in step aa),
and wherein at least a part of the wash phase(s) of c)bb), optionally after separation of catalyst residues and optionally organic solvent residues, is used as partial replacement of the water for producing the sodium hydroxide solution for step b).

## Revendications

1. Procédé de fabrication de diarylcarbonate et traitement d'au moins une partie de la solution contenant du chlorure de métal alcalin en résultant dans une électrolyse de chlorure de métal alcalin intercalée à la suite, comprenant les étapes suivantes:
a) préparation de phosgène en faisant réagir du chlore avec du monoxyde de carbone;
b) transformation du phosgène formé selon l'étape a) par réaction avec au moins un monophénol, en présence d'une base et le cas échéant d'un catalyseur basique, en un diarylcarbonate et en une solution contenant du chlorure de métal alcalin;
c) séparation et traitement du diarylcarbonate formé dans l'étape b);
d) séparation de la solution contenant du chlorure de métal alcalin restante selon l'étape c) des résidus de solvants et le cas échéant des résidus de catalyseur, en particulier par strippage de la solution avec de la vapeur d'eau et traitement par des adsorbants, en particulier par du charbon actif;
e) oxydation électrochimique d'au moins une partie de la solution contenant du chlorure de métal alcalin de l'étape d) avec formation de chlore, de lessive alcaline et le cas échéant, d'hydrogène,
**caractérisé en ce que** lors de la séparation d) de la solution avant le traitement par des adsorbants, le pH de la solution est ajusté à une valeur égale ou inférieure à 8 et
f) au moins une partie du chlore formé selon l'étape e) est recyclée dans la fabrication du phosgène selon l'étape a) et/ou
g) au moins une partie de la lessive alcaline produite selon l'étape e) est recyclée dans la fabrication du diarylcarbonate selon l'étape b).

2. Procédé selon l'étape 1, **caractérisé en ce que** l'oxydation électrochimique d'au moins une partie de la solution contenant du chlorure de métal alcalin de l'étape d) en chlore et en lessive de soude caustique est effectuée en utilisant des électrodes à diffusion de gaz comme cathode.

3. Procédé selon l'étape 1, **caractérisé en ce qu'**au moins une partie de la solution contenant du chlorure de métal alcalin de d) purifiée est introduite dans le circuit de saumure d'une électrolyse à membrane pour la production de chlore, de lessive de soude caustique et, le cas échéant d'hydrogène.

4. Procédé selon l'une des revendications 1 à 3, **caractérisé en que** du chlorure de métal alcalin supplémentaire est additionné à la solution contenant du chlorure de métal alcalin lors de l'électrolyse e), afin d'augmenter la concentration de chlorure de métal alcalin.

5. Procédé selon l'une des revendications 1 à 4, **caractérisé en ce que** le pH de la solution contenant du chlorure de métal alcalin est ajusté lors de la purification d) à une valeur inférieure à 7, en particulier en utilisant de l'acide chlorhydrique ou du chlorure d'hydrogène.

6. Procédé selon l'une des revendications 1 à 5, **caractérisé en que** la concentration de chlorure de métal alcalin de la solution contenant du chlorure de métal alcalin parvenant dans l'électrolyse est comprise entre 100 et 280 g/l, de préférence entre 110 et 220 g/l, et/ou la concentration de la lessive de soude caustique obtenue par l'électrolyse est comprise entre 13 et 33 % en poids, de préférence entre 20 et 32 % en poids.

7. Procédé selon l'une des revendications 1 à 6, **caractérisé en ce que** sont utilisées lors de l'électrolyse e) des membranes échangeuses d'ions dont le transport d'eau par mol de sodium est supérieur à 4 mol d'H₂O par mol de sodium.

8. Procédé selon la revendication 7, **caractérisé en ce que** sont utilisées lors de l'électrolyse e) de préférence des membranes échangeuses d'ions dont le transport d'eau par mol de sodium est compris entre 5,5 et 6,5 mol d'H₂O par mol de sodium.

9. Procédé selon l'une des revendications 1 à 8, **caractérisé en ce que** l'électrolyse est opérée à une densité de courant comprise entre 2 et 6 kA/m², la surface prise pour base pour le calcul de la densité de courant étant la surface de la membrane.

10. Procédé selon l'une des revendications 1 à 9, **caractérisé en ce que** l'électrolyse e) est opérée à une température comprise entre 70 et 100 °C, de préférence entre 80 et 95 °C.

11. Procédé selon l'une des revendications 1 à 10, **caractérisé en ce que** l'électrolyse est opérée à une pression absolue comprise entre 1,0 et 1,4 bar, de préférence entre 1,1 et 1,3 bar.

12. Procédé selon l'une des revendications 1 à 11, **caractérisé en ce que** l'électrolyse e) est opérée avec une pression différentielle entre le compartiment de la cathode et celui de l'anode comprise entre 20 et 150 mbar, de préférence entre 30 et 100 mbar.

13. Procédé selon l'une des revendications 1 à 12, **caractérisé en ce que** l'électrolyse est opérée avec une anode, qui comporte comme revêtement, outre de l'oxyde de ruthénium, également d'autres composés de métaux précieux des 7^{e} et 8^{e} groupes secondaires et/ou du 4^{e} groupe principal du système périodique des éléments.

14. Procédé selon l'une des revendications 1 à 13, **caractérisé en ce que** dans l'électrolyse e) des anodes présentant une surface plus grande que celle des membranes sont utilisées dans les cellules.

15. Procédé selon l'une des revendications 1 à 14, **caractérisé en ce que** la solution contenant du chlorure de métal alcalin de d) est concentrée avant l'électrolyse e) à l'aide d'un procédé à membrane.

16. Procédé selon l'une des revendications 1 à 15, **caractérisé en ce que** sont utilisés comme monophénol dans l'étape b), des alkylphénols, en particulier des crésols, du p-tert.-butylphénol, du p-cumylphénol, du p-n-octylphénol, du p-iso-octylphénol, du p-n-nonylphénol et du p-isononylphénol, des halogénophénols, en particulier du p-chlorophénol, du 2,4-dichlorophénol, du p-bromophénol et du 2,4,6-tribromophénol, avec une préférence particulière du phénol.

17. Procédé selon l'une des revendications 1 à 16, **caractérisé en ce que** l'on utilise la solution contenant du chlorure de métal alcalin des eaux résiduaires de la réaction de b) ou au moins réunie en partie avec l'eau de lavage du traitement c) du diarylcarbonate.

18. Procédé selon l'une des revendications 1 à 17, **caractérisé en ce que** des phénols de la formule (I) 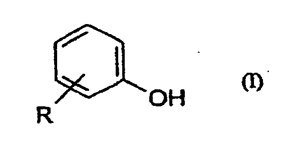 dans laquelle R désigne l'hydrogène, un halogène ou un radical alkyle en C₁ à C₉ ramifié ou linéaire ou un radical alcoxycarbonyle, sont utilisés dans la réaction b).

19. Procédé selon l'une des revendications 1 à 18, **caractérisé en ce que** l'étape c) comprend au moins les étapes suivantes:
aa) séparation de la phase organique contenant le diarylcarbonate et de la solution aqueuse des eaux usées de la réaction contenant du chlorure de métal alcalin;
bb) lavage au moins une seule fois de la phase organique contenant du diarylcarbonate obtenue dans l'étape aa);
et dans lequel au moins une partie de la ou des phases de lavage de l'étape c) bb) est utilisée, le cas échéant après séparation des résidus de catalyseur et le cas échéant des résidus de solvants organique comme succédané partiel de l'eau pour la production de la lessive de soude caustique pour l'étape b).
